# EUROPEAN PATENT SPECIFICATION

(11) **EP 1 641 682 B1**
(45) Date of publication and mention of the grant of the patent: **05.01.2011**
(21) Application number: 04743082.2
(22) Date of filing: 24.06.2004
(51) Int. Cl.: B65D 83/14, A61M 15/00

(54) **PHARMACEUTICAL DISPENSING AID**
VORRICHTUNG ZUM ABGEBEN VON PHARMAZEUTISCHEN PRODUKTEN
DISTRIBUTEUR DE PRODUIT MEDICAMENTEUX

(30) Priority: 24.06.2003 IN MU06582003
(43) Date of publication of application: 05.04.2006
(73) Proprietor: CIPLA LIMITED, Bombay-400008, Maharashtra (IN)
(72) Inventor: MALHOTRA, Geena, Mazgaon, Mumbai 400 010, Maharashtra (IN); LULLA, Amar, Colaba, Mumbai 400 005 Maharashtra (IN)
(74) Representative: Curtis, Philip Anthony
(86) International application number: PCT/GB2004/002731
(87) International publication number: WO 2005/000712

(56) References cited:
- WO-A-96/32345
- GB-A- 2 195 544
- US-A- 3 746 196
- US-A1- 2002 043 262

## Description

This invention in general relates to pharmaceutical dispensing aids. More particularly, this invention relates to an inhaler device for pharmaceuticals, still more particularly to a metered dose inhaler (MDI) for pharmaceuticals.

MDIs are well known in the art. A metered dose inhaler typically comprises a canister crimped with a metering valve, wherein the canister is filled with an aerosol formulation that includes a drug dissolved or dispersed in a propellant. Typically the container of the inhaler is made of jacketed glass or metal. Metallic containers are either made of tin-plated steel or aluminum. The propellants used in metered dose inhalers include chlorofluorocarbons (CFCs), hydrofluorocarbons (HFCs) or hydrofluoroalkanes (HFAs).

CFC propellants are considered to cause ozone depletion. Consequent to the Montreal Protocol and the call for banning of the CFC propellants, new ozone friendly propellants have been developed. These propellants include the HFAs. HFAs do not contain chlorine and arc considered as ozone friendly.

United States Patent Number 6444028, describes a plain aluminum can for an ipratropium formulation.

United States Patent Application Number 20030066525 to Lewis David et al titled 'Pressurized metered dose inhalers (MDI)' discloses a pressurized metered dose inhaler wherein the internal surface of the inhaler consists of stainless steel or anodized aluminum or the internal surface lined with an inert organic coating.

United States Patent Application Number 2002219743 discloses a plastic pressure bottle having a polyethylene terephthalate (PET) layer for alkaline ingredients & manufacture of laminated bottles with outer layer & polyamide inner layer.

Japanese Patent Application No. JP 200020794 discloses a container comprising a mainly PET based layer & a gas barrier layer installed in the center to the inner side of the wall. Thus a five-layered laminate for preparation of container was made from PET as two surfaces & central layer & ethylene-vinyl alcohol copolymer (F10IBZ) as inner layer b/w the central layer & the surface.

It is known from the prior art that canisters for metered dose inhalers may be opaque. They therefore, do not provide the advantage of visible dosage monitoring of the content inside the container. This is especially needed when the canister contains a medicated formulation. The medicament may be in the form of solution or suspension. For suspensions, there exists the possibility of agglomeration. Agglomeration occurs mainly when the medicament is used infrequently or only when the patient's condition demands in such cases, the same medicament is used for months either till it gets over or expires.

A prior art inhaler device and method according to the preamble of the appended independent claims is disclosed in GB 2 195 544 A.

It is possible that due to the non-transparent nature of the container, the patient is unable to estimate the amount of dose remaining in the container. Therefore at the time of an emergency, the medicine may not be available or may be difficult to obtain.

It is also necessary that there be some indication by way of marking or otherwise to the patient about the amount of dosage form remaining in the canister. This can always indicate to the patient that now is the time to buy another canister and keep it in stock.

We have now found a way to enable the patient to visually monitor the form and content of the inhalation medicament contained in the aerosol device. The present invention therefore offers an improved canister, which imparts transparency to the canister.

It has also been observed that if the drug canister is made of polycarbonate, the drug particles of the formulation do not adhere to the inner walls of the polycarbonate container thereby imparting anti-adherent property upon the canister. This also results in giving a better uniformity of dose and content per spray.

In accordance with one embodiment, the present invention provides a pharmaceutical dispensing aid having a transparent canister, which is made of a polycarbonate polymer and does not have any coating on the interior surface thereof. The polycarbonate polymer described herein may be bisphenol A polycarbonate polymer.

In accordance with this embodiment, the present invention provides a pharmaceutical dispensing aid having a transparent canister thus enabling the user thereof to visually monitor the content and form of the medicament contained therein.

In accordance with another embodiment, the present invention provides a pharmaceutical dispensing aid having a transparent canister with etchings or markings that indicate to the patient the level of the medicament inside the container.

The present invention provides a pharmaceutical dispensing aid for administering a medicament, the pharmaceutical dispensing aid having a canister made of polycarbonate polymer wherein the use of polycarbonate polymer prevents adhesion of the medicament to the inner surface of the canister thus enabling uniform dose content per spray.

The present invention provides a pharmaceutical dispensing aid for administering a medicament, the improved pharmaceutical dispensing aid having a canister made up of polycarbonate polymer wherein the use of polycarbonate polymer imparts aesthetic elegance to the container thereby making it useful in various other fields such as cosmetology, nasal sprays, dry powder inhalers, and such other related fields.

The present invention provides a pharmaceutical dispensing aid in the form of a metered dose inhaler device.

One aspect of the present invention provides a metered dose inhaler as recited in the appended independent claim 1.

A further aspect of the present invention provides a method as recited in the appended independent claim 9.

Further features of the present invention are provided as recited in the appended dependent claims.

The present invention describes an improved canister for spraying and inhalation. The canister is made of polycarbonate which makes it transparent.

The preferred purpose of the polycarbonate is to provide the dual function of transparency and reducing adhesion of the formulation to the interior of the dispenser or canister. To this end, the dispenser or canister is preferably made entirely of polycarbonate. It will be appreciated, however, that the transparency function may be achieved in a dispenser or canister which is only partially made of polycarbonate (or another transparent polymer), provided that the transparency is sufficient to permit the interior contents of the dispenser or canister to be visible from the exterior. Thus, a slightly opaque dispenser or canister may be provided, even though a completely transparent canister is preferred. Furthermore, the dispenser or canister may have a generally opaque structure with one or more transparent strips which are sufficient to enable to interior contents of the dispenser or canister to be visible from the exterior.

The transparent container enables the patient to visibly monitor the content of the medicament inside the container. Therefore the patient realizes when the medicament is about to run out, and keeps another canister in stock thereby avoiding situations when the patient is in urgent need of the medication and the medicament is unavailable and difficult to obtain. Transparency of the canister also enables the patient to visualize the physical nature of the medicament. This visualization is critical when the medicament is a suspension or the medicament has not been used since a long time.

For example, before inhalation, the patient shakes the canister and realizes that the drug particles do not disperse completely. In this way, the patient understands that the medicament is unstable and is not fit for inhalation. Therefore transparency of the container becomes the only means that a patient, who is untrained in this field of expertise and the ultimate user of the medicament, realizes that this medicament is not to be inhaled.

The canister may also bear some markings or etchings indicative of the various levels of the content of the canister. Therefore in case of medicated formulations, especially aerosols, the patient can then inhale the doses only in the levels that produces pharmacologically therapeutic effects. Currently the patient has to guess how many doses are left in the canister and has two practical options: (1) throw away the canister that may still contain acceptable metered doses or (2) use a product when it may be beyond the recommended number of doses and risk not receiving correct drug dose. The former is wasteful, and the latter is potentially dangerous.

For example, if the metered dose inhaler is configured to deliver 120 puffs the actual number of puffs filled inside the container is 20 to 30 more than the puffs mentioned on the label. This is because the initial few and the last few puffs do not actually contain the active ingredient in therapeutic amounts. Expulsion of the initial few doses is known as priming of the inhaler, This is necessary so that the next dose the patient inhales has the desired amount of drug in it. The last few puffs actually contain only the propellant which is necessary to expel the active. The etchings or markings are therefore indicative of the levels of the doses that are to be expelled for priming.

Usually the patient is instructed to discard the canister after inhalation of certain number of doses. This reliance on the patient's memory may cause serious casualties because the patient may forget to discard the used canister, that contains only the non-active excipients, and buy a new one. Thus the markings also indicate to the patient the level after which the patient should not use the medicament as it only contains the propellant and no drug.

It has also been observed that the polycarbonate canister prevents adhesion of the active drug particles to the inner walls of the canister. This results in better uniformity of dosage and content per spray with every puff. Therefore the therapeutic performance of the canister is enhanced.

The canister preferably has a cylindrical body having a closed end and a round base and another end having a mouth. The cylindrical body is advantageously made up of polycarbonate polymer. The mouth is preferably crimped with a metering valve of a suitable material such as aluminum or tin. The metering valve may be entirely conventional, as described in the prior art. The capacity of the container may range from 2 ml to 50 ml. The canister may be filled with formulations such as medicated solutions or suspensions for inhalation.

The formulations include one or more active pharmaceutical ingredients. The active pharmaceutical ingredients are typically drugs useful in the treatment of respiratory diseases. The active pharmaceutical ingredients may be selected from a group comprising bronchodilators, beta-2-adrenoacceptors anticholinergics, steroids, beta-2-agonists, antiallergics and such other compounds; including their salts, derivatives, enantiomers, prodrugs and racemic mixtures thereof. The active ingredient is combined with a propellant or a mixture of propellants, preferably selected from the class of HFA propellants. The HFA propellants may be selected from 1,1,1,2-tetrafluoroethane or 1,1,1,2,3,3,3-heptafluoropropane. The formulation may optionally include other pharmaceutically acceptable excipients, such as co-solvents, surfactants and the like. Ethanol may be used as a co-solvent in the range of 1-20 wt% with respect to the formulation. The surfactants may be selected from lecithin, oleic acid, sorbitan triolcate, glycerol and the like, in the range of 0.0001-15 wt% with respect to the active.

The formulation may be provided as a solution of the active pharmaceutical substance in the propellant or as a suspension or dispersion of the active pharmaceutical substance in the propellant.

The transparent polycarbonate canister so designed is aesthetic and elegant and can be used in various other fields such as cosmetology. For cosmoceutical preparations, the canister may be fitted with a continuous valve. The capacity of the canister may range from 10 ml to 500 ml or more as required. The canister may comprise of various cosmetological formulations such as deodorants, hair sprays, hair mousses, air fresheners, shaving creams etc.

The canister may be prepared by any method known in the art. One such suitable illustration of a method of preparation of the said canister is as follows:

An example of a suitable manufacturing process for the polycarbonate container by injection molding / injection blow molding will now be provided.

The polycarbonate needed for manufacturing the can is stored in a silo as pellets. It will be appreciated that the polycarbonate should preferably be free of any opacifying agent, or at least sufficiently free of opacifying agent that the visibility of the formulation within the canister is not impaired. Before use, it is dried at 120°C. The injection-molding machine has a variety of the sections. There is a polycarbonate reservoir. Below is an endless screw powered by an engine, which conveys the polycarbonate to the mold. Resistance heaters melt the plastic by raising it to a temperature of 320°C. The mold has two sections, one fixed & one movable so that the part can be ejected. The mold is cooled by pressurized circulating water with a temperature of 120°C. Polycarbonate granules are loaded into a hopper drier kept at 80°C-90°C so as to remove the moisture. Then these granules are injection molded/injection blow molded (plasticized) at 220-230°C at different zones in the machine.

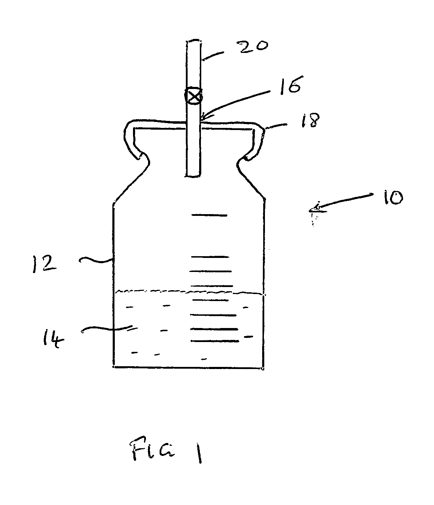

Reference is now made to Figure 1 which is a schematic cross sectional view of a metered dose inhaler according to the present invention. The inhaler is generally designated 10 and comprises a canister 12 which holds a pharmaceutical formulation 14 comprising a drug and a propellant, optionally with excipients. The canister 12 is crimped with a metering valve 16 which comprises a crimped sealing member 18 and a valve member 20. In use, the inhaler 10 would normally be mounted to an actuator (not shown) to which the metering valve. The metering valve 16 and the actuator may be entirely conventional. The canister 12 is entirely made of polycarbonate, whereby the formulation 14 can be seen by the user through the walls of the canister 12. Markings 22 are provided on the canister 12 to give an indication of the number of doses remaining.

### Examples

The following examples of formulations are suitable for use in a metered dose inhaler according to the invention.

### 1. Salbutamol Sulphate HFA4 Inhalation (200 doses).

| Sr. No. | Ingredients | Quantity |
|---|---|---|
| 1. | Salbutamol Sulphate | 28.8 mg |
| 2. | 1,1,1,2-tetrafluroethane | q.s. |

### 2. Ipratropium HFA Inhalation (200 doses)

| Sr. No. | Ingredients | Quantity |
|---|---|---|
| 1. | Ipratropium | 24 mg |
| 2. | 1,1,1,2-tetrafluroethane | q.s. |

### 3. Eudesonide HFA Inhalation (200 doses)

| Sr. No. | Ingredients | Quantity |
|---|---|---|
| 1. | Budesonide | 48 mg |
| 2. | Ethanol | 2.73 gms |
| 3. | Lecithin | 0.24 mg |
| 4. | 1,1,1,2,3,3,3-heptafluoropropane | q.s. |

It will be readily apparent to one skilled in the art that varying substitutions and modifications that fall within the scope of the appended claims, may be made to the invention disclosed herein without departing from the scope of the invention. Thus, it should he understood that although the present invention has been specifically disclosed by preferred embodiments and optional features, modification and variation of the concepts herein disclosed that fall within the scope of the appended claims, may be made by those skilled in the art, and such modifications and variations are considered to fall within the scope of the invention.

## Claims

1. A metered dose inhaler (10) comprising a canister (12) and a metering valve (16) attached to the canister (12), wherein the canister (12) is sufficiently transparent that a formulation (14) disposed within the canister (12) is visible from the exterior of the canister (12), **characterised in that** the canister (12) is made of polycarbonate and does not have any coating on the interior surface thereof.

2. A metered dose inhaler according to claim 1, wherein the canister (12) is entirely transparent.

3. A metered dose inhaler according to claim 1 or 2, wherein the canister (12) is provided with markings indicative of the number of doses of formulation (14) remaining in the canister (12).

4. A metered dose inhaler according to any preceding claim, further comprising a formulation (14) containing an active pharmaceutical substance selected from the group of bronchodilators, long acting bronchodilators, beta-2-adrenoceptors, anticholinergics, steroids, beta-2-agonists and antiallergics.

5. A metered dose inhaler according to claim 4, wherein the active pharmaceutical substance is salbutamol, ipratropium or budesonide.

6. A metered dose inhaler according to claim 4 or 5, wherein the formulation (14) further comprises a propellant.

7. A metered dose inhaler according to any preceding claim, further comprising an actuator for actuating the metering valve (16).

8. A metered dose inhaler according to claim 7, wherein the actuator is configured such that, in use, it does not prevent the user from seeing the level of formulation (14) in the canister (12).

9. A method of making a metered dose inhaler (10), according to any preceding claim, comprising forming a canister (12), placing a pharmaceutical formulation (14) in the canister (12), and then securing a metering valve (16) to the canister (12); **characterised in that** the canister (12) forming step comprises forming a polycarbonate canister (12) by injection molding or injection blow molding.

## Patentansprüche

1. Dosierinhalationsvorrichtung (10), die einen Behälter (12) und ein Dosierventil (16) umfasst, das an dem Behälter (12) angebracht ist, wobei der Behälter (12) ausreichend transparent ist, um eine in dem Behälter (12) befindliche Zubereitung (14) von der Außenseite des Behälters (12) sehen zu können, **dadurch gekennzeichnet, dass** der Behälter (12) aus Polycarbonat besteht und keine Beschichtung an seiner Innenfläche aufweist.

2. Dosierinhalationsvorrichtung nach Anspruch 1, wobei der Behälter (12) vollständig transparent ist.

3. Dosierinhalationsvorrichtung nach Anspruch 1 oder 2, wobei der Behälter (12) mit Markierungen versehen ist, die die Anzahl von Dosen der Zubereitung (14) anzeigen, die in dem Behälter (12) verbleiben.

4. Dosierinhalationsvorrichtung nach einem der vorangehenden Ansprüche, die des Weiteren eine Zubereitung (14) umfasst, die eine aktive pharmazeutische Substanz enthält, die aus der Gruppe von Bronchodilatoren, Langzeit-Bronchodilatoren, β₂-Adrenorezeptoren, Anticholinergika, Steroiden, β₂-Agonisten und Antiallergika ausgewählt wird.

5. Dosierinhalationsvorrichtung nach Anspruch 4, wobei die aktive pharmazeutische Substanz Salbutamol, Ipratropium oder Budesonid ist.

6. Dosierinhalationsvorrichtung nach Anspruch 4 oder 5, wobei die Zubereitung (14) des Weiteren ein Treibmittel umfasst.

7. Dosierinhalationsvorrichtung nach einem der vorangehenden Ansprüche, die des Weiteren ein Betätigungselement zum Betätigen des Dosierventils (16) umfasst.

8. Dosierinhalationsvorrichtung nach Anspruch 7, wobei das Betätigungselement so eingerichtet ist, dass es im Einsatz den Benutzer nicht daran hindert, den Pegel der Zubereitung (14) in dem Behälter (12) zu sehen.

9. Verfahren zum Herstellen einer Dosierinhalationsvorrichtung (10) nach einem der vorangehenden Ansprüche, das Ausbilden eines Behälters (12), Einfüllen einer pharmazeutischen Zubereitung (14) in den Behälter (12) und anschließend Befestigen eines Dosierventils (16) an dem Behälter (12) umfasst, **dadurch gekennzeichnet, dass** der Schritt zum Ausbilden des Behälters (12) Ausbilden eines Polycarbonat-Behälters (12) durch Spritzgießen oder Spritzblasen umfasst.

## Revendications

1. Aérosol doseur (10) comprenant un réservoir (12) et une valve doseuse (16) fixée au réservoir (12), dans lequel le réservoir (12) est suffisamment transparent pour qu'une préparation (14) disposée dans le réservoir (12) soit visible de l'extérieur du réservoir (12), **caractérisé en ce que** le réservoir (12) est constitué de polycarbonate et ne comporte pas de revêtement sur sa surface intérieure.

2. Aérosol doseur selon la revendication 1, dans lequel le réservoir (12) est entièrement transparent.

3. Aérosol doseur selon la revendication 1 ou 2, dans lequel le réservoir (12) est doté de repères indicatifs du nombre de doses de la préparation (14) restant dans le réservoir (12).

4. Aérosol doseur selon l'une quelconque des revendications précédentes, comprenant en outre une préparation (24) contenant une substance pharmaceutique active choisie dans le groupe des broncho-dilatateurs, des broncho-dilatateurs à longue durée d'action, des bêta-2-adrénorécepteurs, les anticholinergiques, des stéroïdes, des bêta-2-agonistes et des anti-allergiques.

5. Aérosol doseur selon la revendication 4, dans lequel la substance pharmaceutique active est le salbutamol, l'ipratropium ou le budésonide.

6. Aérosol doseur selon la revendication 4 ou 5, dans lequel la préparation (24) comprend en outre un agent propulseur.

7. Aérosol doseur selon l'une quelconque des revendications précédentes, comprenant en outre un actionneur pour actionner la valve doseuse (16).

8. Aérosol doseur selon la revendication 7, dans lequel l'actionneur est configuré de telle sorte qu'il n'empêche pas l'utilisateur de voir le niveau de préparation (14) dans le réservoir (12).

9. Procédé de production d'un aérosol doseur (10) selon l'une quelconque des revendications précédentes, comprenant la formation d'un réservoir (12), le placement d'une préparation pharmaceutique (14) dans le réservoir (12) puis la fixation d'une valve doseuse (16) au réservoir (12) ; **caractérisé en ce que** l'étape de formation du réservoir (12) comprend la formation d'un réservoir de polycarbonate (12) par moulage par injection ou moulage par injection - soufflage.
